# FASCICULE DE BREVET EUROPEEN

(11) **EP 2 956 487 B1**
(45) Date de publication et mention de la délivrance du brevet: **24.04.2019**
(21) Numéro de dépôt: 14708633.4
(22) Date de dépôt: 14.02.2014
(51) Int. Cl.: C08F 2/22, C08F 2/38

(54) **UTILISATION DE MERCAPTO-ESTERS METHYLIQUES EN TANT QU'AGENTS DE TRANSFERT DE CHAINE**
VERWENDUNG VON METHYLMERCAPTOESTEN ALS KETTENÜBERTRAGUNGSMITTEL
USE OF METHYL MERCAPTO-ESTERS AS CHAIN TRANSFER AGENTS

(30) Priorité: 15.02.2013 FR 1351312; 15.02.2013 US 201361765058 P
(43) Date de publication de la demande: 23.12.2015
(73) Titulaire: Arkema France, 92700 Colombes (FR)
(72) Inventeur: CAZAUX, Jean-Benoît, 64170 LACQ (FR); SAINT LOUIS AUGUSTIN, Pascal, F-64140 Billere (FR); FREMY, Georges, F-64390 Sauveterre de Bearn (FR)
(86) Numéro de dépôt international: PCT/FR2014/050296
(87) Numéro de publication internationale: WO 2014/125223

(56) Documents cités:
- EP-A1- 0 005 400
- US-A1- 2009 240 034
- A.J. COSTANZA, R.J. COLEMAN, R.M. PIERSON, C.S. MARVEL, C. KING: "Bis-Type Modifiers in Polymerization. II. Comparison of Effectiveness of Various Compounds in Emulsion Butadiene and Bulk Styrene Polymerizations", JOURNAL OF POLYMER SCIENCE, vol. 17, juillet 1955 (1955-07), pages 319-340, XP002699051,
- A.J. COSTANZA, R.J. COLEMAN, R.M. PIERSON, C.S. MARVEL, C. KING: "Bis-Type Modifiers in Polymerization. I. Behavior of Various Disulfides in Bulk styrene Polymerization", JOURNAL OF POLYMER SCIENCE, vol. 17, juillet 1955 (1955-07), pages 221-246, XP002699052,

## Description

L'invention a pour objet des mercapto-esters méthyliques d'acides carboxyliques, et leur procédé de préparation. L'invention concerne également l'utilisation desdits dérivés de mercapto-esters en tant qu'agent de transfert de chaîne dans des réactions de (co)polymérisation radicalaire en émulsion, ainsi que les procédés de (co)polymérisation radicalaire en émulsion mettant en oeuvre lesdits mercapto-esters méthyliques d'acides carboxyliques en tant qu'agents de transfert de chaîne.

Les caractéristiques intrinsèques des polymères telles que la taille moyenne des chaînes et leurs distributions ont de fortes influences sur les propriétés macroscopiques des matériaux résultants. En effet, les propriétés d'un polymère, notamment en solution ou à l'état fondu, telles que la viscosité, sont largement dépendantes de sa masse molaire et de son indice de polymolécularité. En règle générale, plus la masse molaire d'un polymère est faible et de distribution étroite, plus la viscosité dudit polymère sera faible. Une faible viscosité est généralement recherchée dans les applications de mise en forme, par exemple par injection ou extrusion de polymère.

Lorsqu'il est sous forme d'émulsion, de latex et autres, par exemple dans les applications de revêtements et adhésifs, les polymères présentant une étroite distribution en masses molaires sont préférés. De tels polymères à étroite distribution en masses molaires sont généralement préparés en présence d'un agent de transfert de chaîne (ou plus simplement « agent de transfert », ou encore « CTA » acronyme anglais pour « Chain Transfer Agent »).

La mise en oeuvre d'un agent de transfert permet d'obtenir une taille de particules de polymère plus homogène et également une stabilité accrue, en comparaison avec un polymère préparé sans agent de transfert. Cette stabilité accrue permet une meilleure résistance lors des différentes opérations de stockage, de pompage et de transport, et également une compatibilité améliorée des émulsions de polymères, lors des procédés de formulation, avec les composants entrant par exemple dans la composition de peinture.

Les molécules de type mercaptan sont largement utilisées depuis de nombreuses décennies dans l'industrie des polymères en tant qu'agents de transfert de chaîne en polymérisation radicalaire. L'utilisation d'un agent de transfert de type mercaptan en polymérisation radicalaire permet de réduire la taille moyenne des chaînes de polymères, et également, dans certains cas, de réduire leur indice de polymolécularité. Ces agents de transferts peuvent être utilisés dans des polymérisations en masse, en milieux solvantés homogènes ou dispersés.

Cependant, les mercaptans, qui sont des molécules soufrées, peuvent présenter le désavantage d'être odorantes et dans certains cas toxiques. Leurs manutentions et utilisations sont ainsi rendues délicates, nécessitant des mesures spécifiques de protection du personnel et des installations mettant en oeuvre ces composés soufrés, sans compter les législations et règlementations toujours de plus en plus nombreuses qui interdisent l'usage de molécules toxiques et nocives pour l'homme et pour l'environnement.

De nombreuses molécules comportant un groupe mercaptan ont été largement décrites dans la littérature. Par exemple, le brevet US 2,281,613 divulgue l'utilisation d'isohexyl-, d'octadécyl- et de dodécyl-mercaptan en tant qu'agents de transfert lors de la (co)polymérisation en émulsion de 1,3-butadiène et de dérivés de 1,3-butadiène.

Le document US 2,497,107 divulgue l'utilisation de mercapto-ester éthylique d'acide carboxylique dans la copolymérisation en émulsion d'isoprène ou de butadiène en présence d'a-méthylstyrène, de styrène et d'acrylonitrile.

Le brevet US 4,593,081 divulgue l'utilisation de 3-mercaptopropionates d'alkyle dans la copolymérisation en émulsion de monomères acryliques.

Dans Journal of Polymer Science vol. 17, pages 319-340 sont décrits des composés bifonctionnels qui sont utilisés en tant qu'agents de transfert de chaîne en polymérisation en émulsion. Est notamment décrite l'utilisation du sulfure de bis (méthylundécanoate).

Cependant ces agents de transfert de chaîne connus présentent tous un ou plusieurs inconvénients tels que toxicité, nocivité, odeur désagréable, manque d'efficacité, en termes de qualité et de rendement de polymérisation, pour ne citer que les principaux.

Il reste donc un besoin pour des agents de transfert de chaîne ne présentant pas les inconvénients décrits ci-dessus.

De façon surprenante, les inventeurs ont découvert, après diverses expériences et manipulations, que des mercapto-esters méthyliques de certains acides carboxyliques spécifiques, utilisés en tant qu'agents de transfert de chaîne dans les procédés de polymérisation radicalaire en émulsion permettent de préparer des polymères et des copolymères à propriétés améliorées, notamment avec une masse molaire plus faible et une distribution plus étroite que les polymères obtenus avec les mercapto-esters éthyliques d'acides carboxyliques homologues.

Un autre avantage lié à l'utilisation des mercapto-esters méthyliques d'acides carboxyliques selon la présente invention réside dans le fait que ces mercapto-esters peuvent être obtenus à partir de matières premières renouvelables et en particulier à partir de matières premières d'origine végétale.

En outre, ces mercapto-esters qui vont être définis plus loin présentent également l'avantage de ne pas être odorants, voire faiblement odorants, et ne nécessitent pas de précautions particulières lors de leurs utilisations.

Selon un premier aspect, la présente invention concerne ainsi l'utilisation, en tant qu'agent de transfert de chaîne dans un procédé de polymérisation radicalaire en émulsion, d'au moins un mercapto-ester méthylique d'acide carboxylique représenté par la formule (1) suivante : 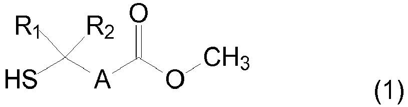 dans laquelle :
- R₁ et R₂, identiques ou différents, sont choisis indépendamment l'un de l'autre parmi l'atome d'hydrogène et un radical hydrocarboné comprenant de 1 à 20 atomes de carbone, linéaire, ramifié ou cyclique, saturé ou insaturé, et éventuellement substitué par un ou plusieurs radicaux choisis parmi carboxy, alkylcarbonyle et alkoxycarbonyle (où alkyle et alkoxy comportent de 1 à 10 atomes de carbone) ; et
- A représente une chaîne divalente hydrocarbonée linéaire, ramifiée ou cyclique, saturé ou insaturé comprenant de 2 à 30 atomes de carbone, bornes incluses, éventuellement interrompue par un ou plusieurs hétéroatomes choisis parmi oxygène, soufre et azote.

Selon un premier mode de réalisation de l'invention, on préfère les composés de formule (1) pour lesquels R₁ et R₂ sont identiques et représentent chacun l'atome d'hydrogène (mercaptans primaires).

Selon un autre mode de réalisation, l'utilisation selon l'invention met en oeuvre au moins un composé de formule (1) dans laquelle R₁ représente l'atome d'hydrogène et R₂ représente un radical hydrocarboné comprenant de 1 à 20 atomes de carbone, linéaire, ramifié ou cyclique, saturé ou insaturé, et éventuellement substitué comme défini précédemment (mercaptans secondaires).

Selon encore un autre mode de réalisation, dans le composé de formule (1), R₁ et R₂, identiques ou différents, représentent chacun un radical hydrocarboné comprenant de 1 à 20 atomes de carbone, linéaire, ramifié ou cyclique, saturé ou insaturé, et éventuellement substitué comme défini précédemment (mercaptans tertiaires).

Dans le cadre de la présente invention, on préfère les mercapto-esters méthyliques d'acides carboxyliques à chaîne mercaptan primaire, puis ceux à chaîne mercaptan secondaire et enfin ceux à chaîne mercaptan tertiaire.

Dans le cas des composés de formule (1) à chaîne mercaptan secondaire ou tertiaire, on préfère ceux pour lesquels les radicaux hydrocarbonés (formant R₂ ou R₁ et R₂ respectivement) sont des radicaux hydrocarbonés comprenant de 1 à 20 atomes de carbone, de préférence de 1 à 10 atomes de carbone, de préférence encore de 1 à 6 atomes de carbone. Les radicaux hydrocarbonés sont choisis parmi les radicaux linéaires, ramifiés ou cycliques, de préférence linéaires, saturés ou insaturés, de préférence saturés.

Parmi les substituants préférés de ces radicaux hydrocarbonés, on peut citer les substituants de type alkoxycarbonyle, où alkoxy comporte de 1 à 10 atomes de carbone, de préférence de 1 à 6 atomes de carbone, de préférence encore de 1 à 4 atomes de carbone, et par exemple alkoxy représente méthyle, éthyle, n-propyle, i-propyle, n-butyle, i-butyle ou t-butyle. Les composés de formule (1) pour lesquels au moins un des radicaux R₁ ou R₂ est substitué par un groupement méthoxycarbonyle sont particulièrement préférés.

Selon un autre mode de réalisation préféré, la chaîne divalente A comporte de 3 à 30 atomes de carbone, de préférence encore de 4 à 30 atomes de carbone, et plus particulièrement de 5 à 30 atomes de carbone, bornes incluses. La chaîne divalente A des composés de formule (1) peut être saturée ou totalement ou partiellement insaturée, linéaire, ramifiée ou cyclique, interrompue par un ou plusieurs hétéroatomes choisis parmi O, S et N, voire être interrompue par un ou plusieurs cycles ou hétérocycles, eux-mêmes saturés ou totalement ou partiellement insaturés. On préfère les chaînes divalentes A qui ne comprennent pas d'hétéroatomes, c'est-à-dire les chaînes divalentes A qui sont des radicaux divalents hydrocarbonés, comportant éventuellement une ou plusieurs insaturations sous forme de double(s) et/ou triple(s) liaison(s).

Des exemples non limitatifs de chaîne divalente A qui peuvent être cités comprennent les chaînes -(CH₂)ₙ-, ou les chaînes -(CH₂)ₙ₁-C=C-(CH₂)ₙ₂-, où n représente un entier compris entre 2 et 30 atomes de carbone, de préférence entre 3 et 30 atomes de carbone, de préférence encore entre 4 et 30 atomes de carbone, et typiquement entre 5 et 30 atomes de carbone, bornes incluses, et n₁ + n₂ est égal à n-2.

Parmi les composés de formule (1), on préfère ceux dans laquelle R₁ et R₂ sont identiques ou différents et représentent chacun un atome hydrogène ou un groupement hydrocarboné et A est tel que défini précédemment.

Selon un mode de réalisation particulièrement préféré, les composés de formule (1) sont ceux pour lesquels A représente un radical divalent hydrocarboné linéaire comprenant de 3 à 18 atomes de carbone, de préférence encore de 4 à 18 atomes de carbone, plus préférentiellement de 6 à 18 atomes de carbone, bornes incluses. Des représentants particulièrement préférés sont ainsi le mercapto-undécanoate de méthyle, le mercaptodécanoate de méthyle, le 9-mercapto-octadécyl-1,18-dioate de diméthyle, de préférence le mercapto-undécanoate de méthyle et le mercaptodécanoate de méthyle, de préférence encore le mercaptodécanoate de méthyle.

Les composés de formule (1) décrits ci-dessus peuvent aisément être préparés à partir de l'ester méthylique insaturé précurseur correspondant qui est engagé dans une réaction de sulfhydratation selon les techniques connues de l'homme du métier. Par « réaction de sulfhydratation », on entend la réaction de Markovnikov d'addition radicalaire de H-S-H sur une double liaison C=C, selon le schéma suivant :

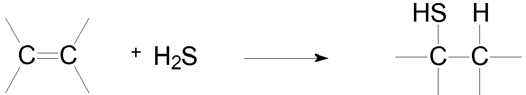

Par ester méthylique insaturé précurseur, on entend un ester méthylique comportant au moins une double liaison susceptible d'être sulfhydratée en une ou deux étapes selon une réaction classique d'addition radicalaire par action du sulfure d'hydrogène (comme décrit par exemple dans FR 2 424 907) ou d'un précurseur de sulfure d'hydrogène, par exemple l'acide thio-acétique (comme décrit par exemple dans US 4 701 492), un mercaptan tertiaire, par exemple le *tert*-butylmercaptan (comme décrit par exemple dans FR 2 603 889), ou par une addition catalytique d'hydrogène sulfuré (comme décrit par exemple dans US 4 102 931).

Ainsi, l'agent de sulfhydratation mise en oeuvre pour la sulfhydratation de l'ester méthylique insaturé en composé de formule (1) peut être de tout type connu de l'homme du métier et par exemple choisi parmi le sulfure d'hydrogène, l'acide thio-acétique (ATA), et autres composés connus de l'homme du métier et habituellement utilisés dans les réactions de sulfhydratation de composés organiques.

Cette réaction de sulfhydratation est avantageusement conduite en présence de catalyseur acide homogène ou hétérogène et/ou sous irradiation de lumière ultra-violette (U.V.) (soit par photolyse directe à des longueurs d'ondes comprises entre 180 nm et 300 nm, soit en présence de photo-initiateurs). Selon un mode de réalisation préféré la réaction de sulfhydratation est conduite sans catalyseur, et sous irradiation U.V.

Cette réaction de sulfhydratation peut être conduite en présence ou en absence de solvant, de préférence en présence d'un ou plusieurs solvants qui peuvent être avantageusement choisis pour leur transparence à la lumière U.V. selon la longueur d'onde utilisée et la facilité de leur séparation du milieu réactionnel. De tels solvants peuvent par exemple être choisis parmi les alcanes légers (1 à 6 atomes de carbone), les éthers d'éthylène-glycol, les hydrocarbures aromatiques, les hydrocarbures aliphatiques, et autres, ainsi que les mélanges de deux ou plusieurs d'entre eux en toutes proportions.

En variante, la réaction de sulfhydratation peut être réalisée en présence d'un ou plusieurs, de préférence un, composé(s) capable(s) de former des radicaux libres. De tels composés sont connus de l'homme du métier et peuvent être choisis par exemple parmi les peroxydes, et de manière indicative et non limitative, parmi le peroxyde d'hydrogène, le peroxyde de sodium ou de potassium, les hydroperoxydes de *tert*-alkyle (par exemple de *tert*-butyle), les peroxydes de *tert*-alkyle, les peresters de *tert*-alkyle, l'hydroperoxyde de cumène, l'azo-bis-*iso-*butyronitrile, et autres, et les mélanges de deux ou plusieurs d'entre eux en toutes proportions.

Lorsque la réaction de sulfhydratation décrite ci-dessus est effectuée par action de l'acide thio-acétique en présence d'un initiateur de radicaux libres et/ou par irradiation à la lumière U.V., comme décrit précédemment, cette réaction est suivie d'une réaction de méthanolyse en milieu acide, permettant de libérer le mercaptan de formule (1) souhaité. Cette réaction de méthanolyse est bien connue et peut être réalisée selon toutes techniques classiques.

À l'issue de l'étape de sulfhydratation, les mercapto-esters peuvent être obtenus sous forme de mélanges d'isomères (mercaptans primaires, secondaires et/ou tertiaires) qui peuvent être ensuite séparés et éventuellement purifiés selon des techniques classiques de séparation et/ou de purification, par exemple par distillation, sous pression atmosphérique ou sous pression réduite selon la nature du mercaptan d'intérêt à récupérer.

Les esters méthyliques précurseurs comportant au moins une double liaison susceptible d'être sulfhydratée par un agent de sulfhydratation sont connus et disponibles dans le commerce ou bien encore peuvent être préparés selon toutes méthodes et modes opératoires connus de l'homme du métier et disponibles dans la littérature brevets, la littérature scientifique, les Chemical Abstracts ou encore l'Internet.

Selon un mode de réalisation, les esters méthyliques précurseurs comportant au moins une double liaison susceptible d'être sulfhydratée peuvent être obtenus par trans-estérification de glycérides (mono-, di- ou triglycérides, de préférence triglycérides) avec du méthanol, selon les méthodes classiques connues de l'homme du métier et par exemple selon le procédé décrit dans EP-B-0 658 183. Les glycérides insaturés qui peuvent être utilisés proviennent essentiellement d'huiles ou de graisses animales ou végétales, de préférence végétales, parmi lesquelles on peut citer à titre indicatif et non limitatif l'huile de soja, l'huile de tournesol, l'huile de lin, l'huile de colza, l'huile de ricin, l'huile de palme, l'huile de palmiste, l'huile de coprah, l'huile de jatropha, l'huile de coton, l'huile d'arachide, l'huile d'olive, l'huile de vernonia, l'huile de cuphéa, l'huile d'hévéa, l'huile de lunaire, l'huile de carthame, l'huile de camélina, l'huile de *Calophyllum inophyllum,* l'huile de *Pongamia pinnata,* le suif de boeuf, l'huile de cuisson ou de la graisse, mais peuvent aussi être des huiles hydrauliques ou de lubrification.

Selon un autre mode de réalisation, les esters méthyliques précurseurs des composés de formule (1) peuvent également être obtenus par métathèse croisée à partir d'autres esters méthyliques, voire de glycérides (ces derniers seront ensuite soumis à une étape de trans-estérification avec du méthanol), tels que par exemple ceux définis précédemment. Les réactions de métathèse sont bien connues de l'homme du métier et font appel le plus souvent à une réaction intermoléculaire entre deux composés porteurs chacun d'au moins une double liaison, comme décrit par exemple dans la demande internationale WO 2009/047444.

En particulier les composés de formule (1) dans lesquels R₁, ou R₁ et R₂, représentent un radical hydrocarboné substitué par un radical alkoxycarbonyle (di- et tri-esters respectivement) peuvent avantageusement être obtenus par métathèse à partir de mono-esters insaturés, selon les techniques de métathèse connues de l'homme du métier. Des exemples d'esters méthyliques insaturés qui peuvent être mis en oeuvre dans une réaction de métathèse pour conduire à des di- et tri-esters comprennent l'oléate de méthyle, le palmitoléate de méthyle, l'arachidonate de méthyle, seuls ou mélanges de deux ou plusieurs d'entre eux en toutes proportions. Selon ce mode de réalisation, le 9-octadécén-1,18-dioate de diméthyle peut par exemple être facilement obtenu par métathèse d'oléate de méthyle et/ou de palmitoléate de méthyle.

Les sources d'esters méthyliques de formule (1) sont ainsi très nombreuses et variées, et des exemples indicatifs et non limitatifs d'esters méthyliques porteurs d'une insaturation susceptible d'être sulfhydratée comprennent, de manière indicative et non limitative les hexénoates de méthyle, les décénoates de méthyle, les undécénoates de méthyle, les dodécénoates de méthyle, l'oléate de méthyle, le linoléate de méthyle, le myristiléate de méthyle, le palmitoléate de méthyle, le linoléate de méthyle, le linolénate de méthyle, l'arachidonate de méthyle, le ricinoléate de méthyle, le 9-octadécén-1,18-dioate de diméthyle, ainsi que les mélanges de deux ou plusieurs d'entre eux en toutes proportions.

De préférence les esters méthyliques insaturés sont choisis parmi les décénoates de méthyle et les undécénoates de méthyle, de préférence encore parmi le décén-9-oate de méthyle et le undécén-10-oate de méthyle.

En variante, les esters méthyliques précurseurs des composés de formule (1) peuvent également être obtenus à partir des acides correspondants, lesquels sont soumis à une réaction d'estérification au méthanol selon les techniques d'estérification classiques bien connues de l'homme du métier.

Des exemples indicatifs et non limitatifs d'acides précurseurs des esters méthyliques comprennent, de manière non limitative les acides hexénoïques, décénoïques, undécénoïques, dodécénoïques, oléique, linoléique, myristique, palmitique, linoléique, linolénique, arachidonique, ricinoléique, les di-acides et tri-acides qui peuvent être obtenus par métathèse croisée selon les méthodes classiques de synthèse par métathèse, comme indiqué ci-dessus, et par exemple le 9-octadécén-1,18-di-oïque. De préférence, lesdits acides sont choisis parmi les décénoïques et les undécénoïques et les mélanges de deux ou plusieurs d'entre eux en toutes proportions, de préférence encore parmi le décén-9-oïque et le undécén-10-oïque.

Parmi les composés de formule (1), on préfère ceux pour lesquels R₁ représente un radical hydrocarboné comprenant de 1 à 20 atomes de carbone, linéaire, ramifié ou cyclique, saturé ou insaturé, substitué par un ou plusieurs radicaux alkoxycarbonyle, où alkoxy comporte de 1 à 10 atomes de carbone et R₂ représente l'hydrogène, d'une part et R₁ et R₂, identiques ou différents représentent chacun un radical hydrocarboné comprenant de 1 à 20 atomes de carbone, linéaire, ramifié ou cyclique, saturé ou insaturé, substitué par un ou plusieurs radicaux alkoxycarbonyle, où alkoxy comporte de 1 à 10 atomes de carbone.

Ainsi et selon un autre aspect, la présente invention concerne les composés de formule (1') suivante : 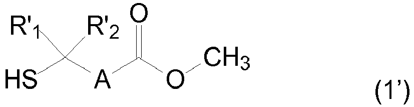 dans laquelle :
- R'₁ représente un radical hydrocarboné comprenant de 1 à 20 atomes de carbone, linéaire, ramifié ou cyclique, saturé ou insaturé, substitué par un ou plusieurs radicaux alkoxycarbonyle, où alkoxy comporte de 1 à 10 atomes de carbone ;
- R'₂ est choisi parmi l'atome d'hydrogène et un radical hydrocarboné comprenant de 1 à 20 atomes de carbone, linéaire, ramifié ou cyclique, saturé ou insaturé, substitué par un ou plusieurs radicaux alkoxycarbonyle, où alkoxy comporte de 1 à 10 atomes de carbone ; et
- A représente une chaîne divalente hydrocarbonée linéaire, ramifiée ou cyclique, saturé ou insaturé comprenant de 2 à 30 atomes de carbone, bornes incluses, éventuellement interrompue par un ou plusieurs hétéroatomes choisis parmi oxygène, soufre et azote.

Les composés de formule (1') forment un sous-ensemble des composés de formule (1), l'ensemble des composés de formule (1') étant compris dans la formule générale (1). On préfère les composés de formule (1') pour lesquels R'₁ représente un radical hydrocarboné comprenant de 1 à 20 atomes de carbone, linéaire, ramifié ou cyclique, saturé ou insaturé, substitué par un radical méthoxycarbonyle, R'₂ représente l'atome d'hydrogène, et A est tel que défini précédemment.

Un exemple de mercaptodiester insaturé de formule (1') est représenté par le 9-mercapto-octadécyl-1,18-dioate de diméthyle.

Selon la présente invention, les mercapto-esters de formule (1) trouvent des applications tout à fait avantageuses dans le domaine de la synthèse de polymères en tant qu'agents de transfert de chaîne.

Ainsi, l'invention concerne également un procédé de (co)polymérisation en émulsion comprenant la formation d'une émulsion dans l'eau d'au moins un monomère avec au moins un mercapto-ester de formule (1) en tant qu'agent de transfert de chaîne.

Plus précisément, l'invention concerne un procédé de (co)polymérisation en émulsion comprenant au moins les étapes suivantes :
a) formation d'une émulsion dans un milieu aqueux d'au moins un monomère à insaturation vinylique,
b) ajout dans ladite émulsion d'au moins un agent de transfert de chaîne de formule (1) tel que défini précédemment,
c) conduite de la réaction de (co)polymérisation, éventuellement en présence d'un amorceur de (co)polymérisation,
d) éventuelle purification et récupération du (co)polymère souhaité.

Dans le procédé de (co)polymérisation selon l'invention, la quantité d'agent(s) de transfert de chaîne de formule (1) est généralement comprise entre 0,01% et 5 % en poids, de préférence entre 0,03% et 3% en poids, par rapport au poids total de l'émulsion.

Quant à la quantité de monomère(s), celle-ci est le plus souvent comprise entre 10% et 60 % en poids, de préférence entre 20% et 50% en poids, par rapport au poids total de l'émulsion.

Les monomères qui peuvent être utilisés dans le procédé de (co)polymérisation selon l'invention sont les monomères couramment et habituellement utilisés dans réaction de polymérisation radicalaire en émulsion, et avantageusement les monomères dits à insaturation(s) vinylique(s), et plus particulièrement les monomères vinyliques, les monomères diéniques conjugués, les monomères acryliques, les monomères méthacryliques, et les mélanges de deux ou plusieurs d'entre eux en toutes proportions, pour ne citer que les plus courants d'entre eux.

Dans un mode de mise en oeuvre du procédé selon l'invention, des exemples non limitatifs de monomères comprennent les acide acrylique, acrylates d'alkyle, acide méthacrylique, méthacrylates d'alkyle, diènes conjugués, styrène et dérivés styréniques, acrylamide, acrylonitrile, ainsi que les mélanges de deux ou plusieurs d'entre eux en toutes proportions.

Selon un mode de réalisation préféré, les monomères sont choisis parmi :
- l'acide acrylique,
- l'acrylate de méthyle, l'acrylate d'éthyle, l'acrylate de n-propyle, l'acrylate d'*iso*-propyle, l'acrylate de n-butyle, l'acrylate d'*iso*-butyle, l'acrylate de *tert*-butyle, l'acrylate de n-pentyle, l'acrylate de *neo*-pentyle, l'acrylate d'*iso*-amyle, l'acrylate de n-hexyle, l'acrylate d'*iso*-hexyle, l'acrylate de cyclohexyle, l'acrylate d'*iso*-octyle, l'acrylate de 2-éthylhexyle, l'acrylate de décyle, l'acrylate d'*iso*-décyle, l'acrylate de lauryle, l'acrylate de stéaryle, et l'acrylate d'*iso*-bornyle, et leurs mélanges,
- l'acide méthacrylique,
- le méthacrylate de méthyle, le méthacrylate d'éthyle, le méthacrylate de n-propyle, le méthacrylate d'*iso*-propyle, le méthacrylate de n-butyle, le méthacrylate d'*iso*-butyle, le méthacrylate de *tert*-butyle, le méthacrylate de n-pentyle, le méthacrylate de *neo*-pentyle, le méthacrylate d'*iso*-amyle, le méthacrylate de n-hexyle, le méthacrylate de d'*iso*-hexyle, le méthacrylate de cyclohexyle, le méthacrylate d'*iso*-octyle, le méthacrylate de 2-éthylhexyle, le méthacrylate de décyle, le méthacrylate d'*iso*-décyle, le méthacrylate de lauryle, le méthacrylate de stéaryle, le méthacrylate d'hydroxy-éthyle, le méthacrylate d'hydroxypropyle, le méthacrylate d'acétoxy-éthyle, le méthacrylate d'acétoxypropyle, le méthacrylate de *tert*-butylamino-éthyle, le méthacrylate de 2-(3-oxazolidinyl)éthyle, le méthacrylate d'*iso*-bornyle, et autres et leurs mélanges,
- l'acrylonitrile, le méthacrylonitrile, l'acrylamide, le méthacrylamide, et leurs mélanges,
- les allylacétoacétates, l'éthylène, le propylène, le styrène et les styrènes substitués, le butadiène, l'acétate de vinyle, le versatate de vinyle, les butyrates de vinyle et autres esters de vinyle, les monomères vinyliques halogénés, tels que le chlorure de vinyle, le chlorure de vinylidène, et autres et leurs mélanges.

Les composés monomères listés ci-dessus peuvent bien entendu être utilisés seuls ou en mélanges, par exemple en mélanges de deux ou plusieurs d'entre eux en toutes proportions. Lorsque deux ou plusieurs monomères sont mis en oeuvre dans le procédé de la présente invention, il en résulte un copolymère, lorsqu'un seul monomère est mis en oeuvre dans le procédé de la présente invention, il en résulte un homopolymère. Les copolymères et homopolymères sont regroupés sous le terme générique de (co)polymères dans la description de la présente invention.

Les copolymères peuvent être obtenus sous forme de copolymères aléatoires, à blocs, séquencés ou alternés, selon les conditions opératoires du procédé de copolymérisation, conditions qui bien connues de l'homme du métier.

Le milieu réactionnel de (co)polymérisation peut également comprendre au moins un tensio-actif et/ou au moins un amorceur de polymérisation et/ou au moins un agent de terminaison de chaîne.

Dans un mode de réalisation de la présente invention, le ou les tensio-actif(s) utilisé(s) peuvent être choisi(s) parmi les tensio-actif(s)anioniques et/ou les tensio-actif(s) non-ioniques, tels que les sulfates, les sulfonates ou les phosphates d'alkyle, d'aryle ou d'alkylaryle de métaux alcalins ou les sels d'ammonium correspondants. Des tensio-actifs tels que les acides alkylsulfoniques, les sels de sulfosuccinates, les sels d'acides gras, les alcools éthoxylés, les copolymères amphiphiles, ainsi que les mélanges de deux ou plusieurs d'entre eux, peuvent être également utilisés.

De manière générale, la quantité de tensio-actif(s) dépend de la concentration en monomère(s) dans l'émulsion, de la nature du ou des monomères, et le plus souvent est comprise entre 0,05% et 10% en poids par rapport au poids total de l'émulsion.

La (co)polymérisation en émulsion de monomères selon l'invention peut être amorcée par tout type d'amorceurs radicalaires, généralement de type oxydant, connus de l'homme du métier, tels que, de manière non limitative, le peroxyde d'hydrogène, le peroxyde de sodium ou de potassium, l'hydroperoxyde de *tert*-butyle et les hydroperoxydes de *tert*-alkyle en général, les peroxydes de *tert*-alkyle, les peresters de *tert*-alkyle, l'hydroperoxyde de cumène, les persulfates d'ammonium ou de métaux alcalins, les perborates de métaux alcalins (par exemple perborate de sodium), l'acide perphosphorique et ses sels associés, le permanganate de potassium, les sels d'ammonium ou de métaux alcalins d'acides peroxydisulfuriques, ainsi que les mélanges de deux ou plusieurs d'entre eux.

Pour un amorçage de la réaction par oxydoréduction, au moins oxydant choisi parmi ceux listés ci-dessus peut-être utilisé en association avec un agent réducteur tel que l'acide ascorbique, l'acide iso-ascorbique, le formaldéhyde sulfoxylé de sodium, le sulfite de sodium, le bisulfite de sodium, le thiosulfate de sodium, l'hydrosulfite de sodium, le sulfure de sodium, l'hydrosulfure ou le dithiosulfate de sodium, l'acide formamidinesulfonique, l'acide hydroxyméthanesulfonique, le 2-hydroxy-2-sulfinatoacétate de sodium, le bisulfite d'acétone, l'éthanolamine, l'acide glycolique, l'acide lactique, l'acide glycérique, l'acide malique, et l'acide tartrique, ainsi que les mélanges de deux ou plusieurs d'entre eux.

Les réactions d'oxydoréduction peuvent elles-mêmes être catalysées par des sels de métaux tels que les sels de fer, cuivre, manganèse, argent, platine, vanadium, nickel, chrome, palladium ou cobalt. Ces catalyseurs peuvent être ajoutés à des teneurs comprises entre 0,01 ppm et 25 ppm en poids par rapport au poids total de l'émulsion.

La quantité d'amorceur(s) de (co)polymérisation est généralement comprise entre 0,001 % et 1% en poids par rapport au poids total de l'émulsion.

La phase émulsionnante est généralement de l'eau ou un mélange eau/solvant(s) organique(s) dont la quantité est celle suffisante pour (q.s.p.) atteindre 100% en poids par rapport au poids total de l'émulsion.

Dans un mode de réalisation du procédé, l'émulsion de monomère(s) comprend entre 0,1% et 1% en poids d'au moins un composé de formule (1), entre 20% et 60% en poids d'au moins un monomère à insaturation(s) vinylique(s) défini précédemment, entre 0,1% et 5% en poids d'au moins un tensio-actif, entre 0,005% et 1% en poids d'au moins un amorceur de polymérisation, et de l'eau en quantité suffisante pour atteindre 100% en poids par rapport au poids total de l'émulsion.

Selon un mode de réalisation du procédé de l'invention, le composé de formule (1) peut être ajouté en continu ou en discontinu, soit en début de réaction, soit en plusieurs fois au cours du procédé, ou bien en continu tout au long du procédé ou tout au long d'une partie du procédé.

Les inventeurs ont découvert de manière surprenante que la réaction de (co)polymérisation radicalaire est d'autant mieux contrôlée, en termes de masse molaire et de polymolécularité, qu'elle est conduite en présence d'au moins un agent de transfert de formule (1), c'est-à-dire d'au moins un mercapto-ester méthylique, où la fonction mercaptan est tertiaire, secondaire, ou primaire, de préférence secondaire ou primaire, de préférence encore primaire.

L'émulsion selon l'invention peut-être préparée selon les techniques usuelles bien connues de l'homme du métier du domaine des émulsions de monomères. Le procédé en émulsion selon l'invention peut être conduit soit en batch soit en continu, et soit avec un ajout unique de monomère(s) en début de réaction soit avec un ou plusieurs ajout(s) continu(s) ou discontinu(s) de monomère(s) au cours du temps, selon les techniques couramment utilisés dans le domaine des émulsions.

De même, le(s) amorceur(s) de polymérisation peut(peuvent) être ajouté(s) en une seule fois en début de procédé, en plusieurs fois au cours du procédé, ou bien en continu tout au long du procédé.

La phase d'initiation de la réaction de (co)polymérisation radicalaire peut être conduite par voie thermique, photochimique, électrochimique ou par réaction d'oxydoréduction, ou encore par toute méthode connue de l'homme du métier spécialiste de ce type de réaction.

La réaction d'amorçage de la polymérisation par voie thermique est préférentiellement réalisée à une température comprise entre 50°C et 100°C.

La réaction de (co)polymérisation selon la présente invention peut-elle-même être conduite à toute température et toute pression que l'homme du métier saura adapter en fonction de la nature et de la quantité/concentration des monomères présents dans l'émulsion. De manière avantageuse, le procédé de (co)polymérisation de l'invention est conduit à pression atmosphérique à une température comprise entre 0°C et 100°C, de préférence entre 10°C et 90°C.

Les exemples suivants, non limitatifs, permettent d'illustrer et de mieux comprendre l'invention.

### Exemple 1 : Préparation de 10-mercaptodécanoate de méthyle

De l'acide 9-décénoïque (100g; 0,588 mol) est ajouté à 237 g (300 mL; 7,406 mol) de méthanol et l'ensemble est porté à reflux, sous agitation en présence de 10 g de résine échangeuse de cations Amberlyst® 15 humide. Le montage utilise un séchage en continu du méthanol par passage sur un Soxhlet chargé de tamis moléculaire (30 g). Après 24 heures au reflux et élimination du solvant sous vide, le taux de conversion de l'acide en ester est voisin de 99%.

Le 9-décénoate de méthyle ainsi obtenu (156 g) est introduit dans un réacteur photochimique comprenant une boucle réactionnelle, avec 100 g de pentane et 60 équivalents molaires de sulfure d'hydrogène liquéfié (1806 g condensés à 20°C sous une pression de 17,5 bars).

Le mélange est recirculé (60 L/h) dans la boucle réactionnelle au sein de laquelle il est soumis à des radiations U.V. (longueur d'onde : 254 nm, puissance: 12 Watt) pendant 3 heures à une température de 38°C, et une pression de 23 bars.

L'excès de sulfure d'hydrogène est ensuite purgé vers un oxydateur thermique par décompression du milieu, puis par un strippage à l'azote. Le mélange est ensuite distillé afin d'éliminer le solvant et les sulfures formés. (T : 130°C, Pression : 5 mbar).

Le mercaptan ainsi obtenu présente une pureté supérieure à 98,5% (mesurée par chromatographie). La quantité de mercaptan primaire obtenu est de 97,7%, et la quantité de mercaptan secondaire obtenu est de 2,3%, ces pourcentages étant des pourcentages en poids par rapport au poids total de mercaptans obtenus.

### Exemple 2 : Préparation de 11-mercapto-undécanoate de méthyle

Le 11-mercapto-undécanoate de méthyle est préparé selon un mode opératoire similaire à celui décrit dans l'exemple 1 ci-dessus, à partir du 10-undecénoate de méthyle.

### Exemples 3 à 8: Polymérisation en émulsion

Dans un réacteur double enveloppe réfrigéré, équipé d'un moteur d'agitation et d'un condenseur à reflux, sont ajoutés 40 g d'acrylate de butyle (Aldrich), 40 g de méthacrylate de méthyle (Arkema), 0,04 g de persulfate de potassium (Aldrich), 2,4 g de tensio-actif Dowfax2A1 (Dow), 0,04 g d'hydrogénocarbonate de sodium (tampon, Aldrich), 332 g d'eau déminéralisée et 0,4 g d'agent de transfert de chaîne selon la présente invention.

L'émulsion de monomère est chauffée à une température comprise entre 70°C et 75°C pendant 5 heures.

La réaction de polymérisation est stoppée lorsque le taux en extrait sec atteint 20% en poids. Le taux en extrait sec est mesuré au moyen d'une thermobalance Mettler à la température de 105°C.

L'analyse des polymères par chromatographie d'exclusion stérique est réalisée dans le tétrahydrofurane (THF) à 40°C à 0,3 g/L, avec un débit de 1 mL/min, sur un jeu de colonne de perméation sur gel (Plgel) MIXED A (30 cm) avec un détecteur réfractométrique. Les colonnes sont étalonnées avec un standard de poly(méthacrylate de méthyle).

Cette analyse par chromatographie d'exclusion stérique permet de déterminer la masse moléculaire moyenne en poids (Mw), ainsi que la masse moléculaire moyenne en nombre (Mn). L'indice de polymolécularité est obtenu en calculant le rapport Mw/Mn. Les résultats sont présentés dans le Tableau 1 suivant :

**-- Tableau 1 --**

| ***Exemple*** | ***Agent de transfert de chaîne*** | ***Masse molaire (Mw) (kg*/*mol)*** | ***Indice de polymolécularité*** |
|---|---|---|---|
| 3 | 11-mercapto-undécanoate de méthyle | 129 | 2,6 |
| 4 | 10-mercaptodécanoate de méthyle | 105 | 2,5 |
| 5* | n-dodécylmercaptan | 140 | 4 |
| 6* | *tert*-dodécylmercaptan | 381 | 9,3 |
| 7* | 11-mercapto-undécanoate d'éthyle | 161 | 3 |
| 8* | acide mercapto-undécanoïque | 1200 | 4,4 |

| | | | |
|---|---|---|---|
| ** indique les exemples comparatifs* | | | |

Les exemples 3 à 8 ci-dessus sont des exemples de préparation de copolymère acrylate de butyle/méthacrylate de méthyle avec des conditions opératoires identiques, exception faite des agents de transfert utilisés.

Les copolymérisations des exemples 3 et 4 sont réalisées avec les agents de transfert de chaîne selon l'invention. Dans les exemples 5 et 6 (comparatifs), les agents de transferts de chaîne sont des mercaptans à chaîne linéaire et ramifiée respectivement et sont disponibles chez Arkema. Dans l'exemple 7 (comparatif), l'agent de transfert de chaîne a été préparé selon un mode opératoire similaire à celui utilisé pour la préparation des agents de transfert de chaîne selon l'invention à partir d'acide 10-décénoïque soumis à une réaction d'estérification avec de l'éthanol. Enfin dans l'exemple 8 (comparatif) l'agent de transfert utilisé est un mercapto-acide disponible auprès d'Aldrich.

Les résultats du Tableau 1 ci-dessus montrent que les agents de transfert de chaîne selon la présente invention sont particulièrement bien adaptés pour la préparation de (co)polymères en émulsion. En effet, les (co)polymères obtenus présentent non seulement des tailles (Mw) tout à fait conformes à celles des copolymères obtenus avec des agents de transfert de chaîne classiquement utilisés dans l'art antérieur, voir des tailles inférieures, mais aussi et surtout présentent des indices de polymolécularité très faibles, et significativement inférieurs à ceux obtenus avec les agents de transfert de chaîne de type mercapto-esters éthyliques, pourtant structurellement proches.

L'utilisation d'agents de transfert de chaîne selon l'invention permet ainsi la préparation de (co)polymères dont les propriétés en termes de poids moléculaire et de polymolécularité sont tout à fait inattendues au regard de l'art antérieur. Ceci permet d'envisager l'utilisation de ces (co)polymères dans des applications spécifiques nécessitant des polymères à faible indice de polymolécularité, par exemple dans les domaines des peintures et des revêtements en général, des adhésifs, du couchage du papier, des additifs de lubrification et autres.

## Revendications

1. Utilisation, en tant qu'agent de transfert de chaîne dans un procédé de polymérisation radicalaire en émulsion, d'au moins un mercapto-ester méthylique d'acide carboxylique de formule (1) : 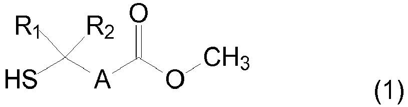 dans laquelle :
- R₁ et R₂, identiques ou différents, sont choisis indépendamment l'un de l'autre parmi l'atome d'hydrogène et un radical hydrocarboné comprenant de 1 à 20 atomes de carbone, linéaire, ramifié ou cyclique, saturé ou insaturé, et éventuellement substitué par un ou plusieurs radicaux choisis parmi carboxy, alkylcarbonyle et alkoxycarbonyle (où alkyle et alkoxy comportent de 1 à 10 atomes de carbone) ; et
- A représente une chaîne divalente hydrocarbonée linéaire, ramifiée ou cyclique, saturé ou insaturé comprenant de 2 à 30 atomes de carbone, bornes incluses, éventuellement interrompue par un ou plusieurs hétéroatomes choisis parmi oxygène, soufre et azote.

2. Utilisation d'un composé selon la revendication 1, dans laquelle R₁ et R₂ représentent chacun l'atome hydrogène.

3. Utilisation d'un composé selon la revendication 1 ou la revendication 2, dans laquelle A est une chaîne -(CH₂)ₙ-, ou une chaîne -(CH₂)ₙ₁-C=C-(CH₂)ₙ₂-, où n représente un entier compris entre 2 et 30 atomes de carbone, de préférence entre 3 et 30 atomes de carbone, de préférence encore entre 4 et 30 atomes de carbone, et typiquement entre 5 et 30 atomes de carbone, bornes incluses, et n₁ + n₂ est égal à n-2.

4. Utilisation selon l'une quelconque des revendications précédentes, dans lequel le composé de formule (1) est le mercapto-undécanoate de méthyle ou le mercaptodécanoate de méthyle, de préférence le mercaptodécanoate de méthyle.

5. Composé de formule (1') : 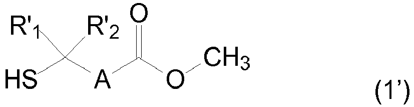 dans laquelle :
- R'₁ représente un radical hydrocarboné comprenant de 1 à 20 atomes de carbone, linéaire, ramifié ou cyclique, saturé ou insaturé, substitué par un ou plusieurs radicaux alkoxycarbonyle, où alkoxy comporte de 1 à 10 atomes de carbone ;
- R'₂ est choisi parmi l'atome d'hydrogène et un radical hydrocarboné comprenant de 1 à 20 atomes de carbone, linéaire, ramifié ou cyclique, saturé ou insaturé, substitué par un ou plusieurs radicaux alkoxycarbonyle, où alkoxy comporte de 1 à 10 atomes de carbone ; et
- A représente une chaîne divalente hydrocarbonée linéaire, ramifiée ou cyclique, saturé ou insaturé comprenant de 2 à 30 atomes de carbone, bornes incluses, éventuellement interrompue par un ou plusieurs hétéroatomes choisis parmi oxygène, soufre et azote.

6. Composé selon la revendication 5, dans lequel R'₁ représente un radical hydrocarboné comprenant de 1 à 20 atomes de carbone, linéaire, ramifié ou cyclique, saturé ou insaturé, substitué par un radical méthoxycarbonyle, R'₂ représente l'atome d'hydrogène, et A est tel que défini dans la revendication 5.

7. Composé selon la revendication 5 ou la revendication 6 qui est le 9-mercapto-octadécyl-1,18-dioate de diméthyle.

8. Procédé de (co)polymérisation en émulsion comprenant au moins les étapes suivantes :
a) formation d'une émulsion dans un milieu aqueux d'au moins un monomère à insaturation vinylique,
b) ajout dans ladite émulsion d'au moins un agent de transfert de chaîne de formule (1) selon l'une quelconque des revendications 5 à 7,
c) conduite de la réaction de (co)polymérisation, éventuellement en présence d'un amorceur de (co)polymérisation,
d) éventuelle purification et récupération du (co)polymère souhaité.

9. Procédé selon la revendication 8, dans lequel la quantité d'agent(s) de transfert de chaîne de formule (1) est comprise entre 0,01% et 5 % en poids, de préférence entre 0,03% et 3% en poids, par rapport au poids total de l'émulsion.

10. Procédé selon la revendication 8 ou la revendication 9, dans lequel les monomères à insaturation vinylique sont les monomères vinyliques, les monomères diéniques conjugués, les monomères acryliques, les monomères méthacryliques, et les mélanges de deux ou plusieurs d'entre eux en toutes proportions.

11. Procédé selon l'une quelconque des revendications 8 à 10, dans lequel le milieu réactionnel comprend en outre au moins un tensio-actif et/ou au moins un amorceur de polymérisation et/ou au moins un agent de terminaison de chaîne.

## Patentansprüche

1. Verwendung mindestens eines Mercaptocarbonsäuremethylesters der Formel (1): 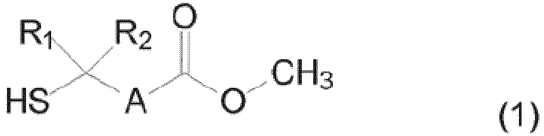 als Kettenübertragungsmittel bei einem Verfahren der radikalischen Emulsionspolymerisation, wobei:
- R₁ und R₂ gleich oder verschieden sind und unabhängig voneinander aus einem Wasserstoffatom und einem linearen, verzweigten oder cyclischen, gesättigten oder ungesättigten Kohlenwasserstoffrest mit 1 bis 20 Kohlenstoffatomen, der gegebenenfalls durch einen oder mehrere Reste, die aus Carboxy, Alkylcarbonyl und Alkoxycarbonyl (wobei Alkyl und Alkoxy 1 bis 10 Kohlenstoffatome aufweisen) ausgewählt sind, substituiert ist, ausgewählt sind; und
- A für eine lineare, verzweigte oder cyclische, gesättigte oder ungesättigte zweiwertige Kohlenwasserstoffkette mit 2 bis 30 Kohlenstoffatomen einschließlich der Grenzen, die gegebenenfalls durch ein oder mehrere Heteroatome, die aus Sauerstoff, Schwefel und Stickstoff ausgewählt sind, unterbrochen ist, steht.

2. Verwendung einer Zusammensetzung nach Anspruch 1, wobei R₁ und R₂ jeweils für ein Wasserstoffatom stehen.

3. Verwendung einer Verbindung nach Anspruch 1 oder Anspruch 2, wobei A für eine -(CH₂)ₙ-Kette oder eine - (CH₂)ₙ₁-C=C-(CH₂)ₙ₂-Kette steht, wobei n für eine ganze Zahl zwischen 2 und 30 Kohlenstoffatomen, vorzugsweise zwischen 3 und 30 Kohlenstoffatomen, noch weiter bevorzugt zwischen 4 und 30 Kohlenstoffatomen und typischerweise zwischen 5 und 30 Kohlenstoffatomen einschließlich der Grenzen, steht und n₁ + n₂ gleich n-2 ist.

4. Verwendung nach einem der vorhergehenden Ansprüche, wobei es sich bei der Verbindung der Formel (1) um Mercaptoundecansäuremethylester oder Mercaptodecansäuremethylester, vorzugsweise Mercaptodecansäuremethylester, handelt.

5. Verbindung der Formel (1'): 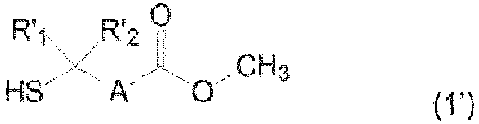 wobei:
- R'₁ für einen linearen, verzweigten oder cyclischen, gesättigten oder ungesättigten Kohlenwasserstoffrest mit 1 bis 20 Kohlenstoffatomen, der gegebenenfalls durch einen oder mehrere Alkoxycarbonylreste, wobei Alkoxy 1 bis 10 Kohlenstoffatome aufweist, substituiert ist, steht;
- R'₂ aus einem Wasserstoffatom und einem linearen, verzweigten oder cyclischen, gesättigten oder ungesättigten Kohlenwasserstoffrest mit 1 bis 20 Kohlenstoffatomen, der gegebenenfalls durch einen oder mehrere Alkoxycarbonylreste, wobei Alkoxy 1 bis 10 Kohlenstoffatome aufweist, substituiert ist, ausgewählt ist;
- A für eine lineare, verzweigte oder cyclische, gesättigte oder ungesättigte zweiwertige Kohlenwasserstoffkette mit 2 bis 30 Kohlenstoffatomen einschließlich der Grenzen, die gegebenenfalls durch ein oder mehrere Heteroatome, die aus Sauerstoff, Schwefel und Stickstoff ausgewählt sind, unterbrochen ist, steht.

6. Verbindung nach Anspruch 5, wobei R'₁ für einen linearen, verzweigten oder cyclischen, gesättigten oder ungesättigten Kohlenwasserstoffrest mit 1 bis 20 Kohlenstoffatomen, der durch einen Methoxycarbonylrest substituiert ist, steht, R'₂ für ein Wasserstoffatom steht und A wie in Anspruch 5 definiert ist.

7. Verbindung nach Anspruch 5 oder Anspruch 6, bei der es sich um 9-Mercaptooctadecyl-1,18-disäuremethylester handelt.

8. Emulsions(co)polymerisationsverfahren, das mindestens die folgenden Schritte umfasst:
a) Bilden einer Emulsion mindestens eines vinylisch ungesättigten Monomers in einem wässrigen Medium,
b) Zugeben von mindestens einem Kettenübertragungsmittel der Formel (1) nach einem der Ansprüche 5 bis 7 zu der Emulsion,
c) Durchführen der (Co)polymerisationsreaktion, gegebenenfalls in Gegenwart eines (Co)polymerisationsinitiators,
d) gegebenenfalls Reinigen und Gewinnen des gewünschten (Co)polymers.

9. Verfahren nach Anspruch 8, wobei die Menge an Kettenübertragungsmittel(n) der Formel (1) zwischen 0,01 und 5 Gew.-%, vorzugsweise zwischen 0,03 und 3 Gew.-%, bezogen auf das Gesamtgewicht der Emulsion, liegt.

10. Verfahren nach Anspruch 8 oder Anspruch 9, wobei es sich bei den vinylisch ungesättigten Monomeren um Vinylmonomere, konjugierte Dienmonomere, Acrylmonomere, Methacrylmonomere und Mischungen von zwei oder mehr davon in beliebigen Anteilen handelt.

11. Verfahren nach einem der Ansprüche 8 bis 10, wobei das Reaktionsmedium außerdem mindestens ein Tensid und/oder mindestens einen Polymerisationsinitiator und/oder mindestens ein Kettenabbruchmittel umfasst.

## Claims

1. Use, as a chain transfer agent in a free-radical emulsion polymerization process, of at least one mercapto-methyl ester of a carboxylic acid, of formula (1): 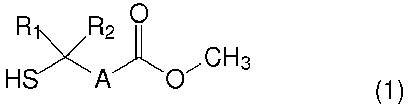 in which:
- R₁ and R₂, which may be identical or different, are chosen, independently of one another, from a hydrogen atom and a linear, branched or cyclic, saturated or unsaturated hydrocarbon-based radical comprising from 1 to 20 carbon atoms, and optionally substituted with one or more radicals chosen from carboxy, alkylcarbonyl and alkoxycarbonyl (where alkyl and alkoxy comprise from 1 to 10 carbon atoms); and
- A represents a linear, branched or cyclic, saturated or unsaturated, hydrocarbon-based divalent chain comprising from 2 to 30 carbon atoms, limits included, optionally interrupted with one or more heteroatoms chosen from oxygen, sulfur and nitrogen.

2. Use of a compound according to Claim 1, in which R₁ and R₂ each represent a hydrogen atom.

3. Use of a compound according to Claim 1 or Claim 2, in which A is a -(CH₂)ₙ- chain, or a -(CH₂)ₙ₁-C=C-(CH₂)ₙ₂- chain, where n represents an integer between 2 and 30 carbon atoms, preferably between 3 and 30 carbon atoms, more preferably between 4 and 30 carbon atoms, and typically between 5 and 30 carbon atoms, limits included, and n₁ + n₂ is equal to n-2.

4. Use according to any one of the preceding claims, in which the compound of formula (1) is methyl mercaptoundecanoate or methyl mercaptodecanoate, preferably methyl mercaptodecanoate.

5. Compound of formula (1'): 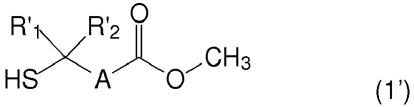 in which:
- R'₁ represents a linear, branched or cyclic, saturated or unsaturated hydrocarbon-based radical comprising from 1 to 20 carbon atoms, substituted with one or more alkoxycarbonyl radicals, where alkoxy comprises from 1 to 10 carbon atoms;
- R'₂ is chosen from a hydrogen atom and a linear, branched or cyclic, saturated or unsaturated hydrocarbon-based radical comprising from 1 to 20 carbon atoms, substituted with one or more alkoxycarbonyl radicals, where alkoxy comprises from 1 to 10 carbon atoms; and
- A represents a linear, branched or cyclic, saturated or unsaturated, hydrocarbon-based divalent chain comprising from 2 to 30 carbon atoms, limits included, optionally interrupted with one or more heteroatoms chosen from oxygen, sulfur and nitrogen.

6. Compound according to Claim 5, in which R'₁ represents a linear, branched or cyclic, saturated or unsaturated hydrocarbon-based radical comprising from 1 to 20 carbon atoms, substituted with a methoxycarbonyl radical, R'₂ represents a hydrogen atom, and A is as defined in Claim 5.

7. Compound according to Claim 5 or Claim 6, which is dimethyl 9-mercaptooctadecyl-1,18-dioate.

8. Emulsion (co)polymerization process comprising at least the following steps:
a) forming an emulsion, in an aqueous medium, of at least one monomer containing vinyl unsaturation,
b) adding to said emulsion at least one chain transfer agent of formula (1) according to any one of Claims 5 to 7,
c) carrying out the (co)polymerization reaction, optionally in the presence of a (co)polymerization initiator,
d) optionally purifying and recovering the desired (co)polymer.

9. Process according to Claim 8, in which the amount of chain transfer agent(s) of formula (1) is between 0.01% and 5% by weight, preferably between 0.03% and 3% by weight, relative to the total weight of the emulsion.

10. Process according to Claim 8 or Claim 9, in which the monomers containing vinyl unsaturation are vinyl monomers, conjugated diene monomers, acrylic monomers, methacrylic monomers, and mixtures of two or more of them in any proportions.

11. Process according to any one of Claims 8 to 10, in which the reaction medium also comprises at least one surfactant and/or at least one polymerization initiator and/or at least one chain-terminating agent.
